# EUROPEAN PATENT APPLICATION

(11) **EP 4 000 528 A1**
(43) Date of publication of application: **25.05.2022**
(21) Application number: 21154410.1
(22) Date of filing: 29.01.2021
(51) Int. Cl.: A61B 7/00, A61B 7/04, A61B 5/00, A61B 5/11

(54) **METHOD FOR RECOGNIZING PHYSIOLOGICAL SYMPTOM AND PHYSIOLOGICAL SYMPTOM SENSING SYSTEM**

(30) Priority: 19.11.2020 TW 109140487
(71) Applicant: Wistron Corporation, New Taipei City 22181 (TW)
(72) Inventor: Huang, Guan-Lin, 22181 New Taipei City (TW)
(74) Representative: 2K Patentanwälte Blasberg Kewitz & Reichel

(57) **Abstract**

A method for recognizing physiological symptom and a physiological symptom sensing system are provided. After the sound signal is obtained, multiple signal segments are captured from the sound signal. Afterwards, a sound type corresponding to each signal segment is recognized. Moreover, a physiological state of a living body is determined based on a plurality of the sound type of the signal segments and combinations of a plurality of the sound types.

## Description

### BACKGROUND

### Field of the Disclosure

The disclosure relates to a sound processing mechanism, and more particularly to a method for recognizing physiological symptoms and a physiological symptom sensing system.

### Description of Related Art

Different generations have different lifestyles or backgrounds, and people confront gastrointestinal problems regardless of age. The gastrointestinal tract is like the main arterial road in the city, serving as one of the paths which human waste and bad substances always flow through. When bad substances stay in the body for a long time, the function of the gastrointestinal tract will deteriorate, which may cause abdominal discomfort or inflammation. There are lots of people who suffer from gastrointestinal and digestive system problems each year. However, there is a lack of awareness of gastrointestinal disorders, people tend to rely on over-the-counter medicines, or mistakenly believe that their gastrointestinal disorders are simply short-term symptoms and thus making no attempt to seek medical treatment. Therefore, the long established habits eventually lead to gastrointestinal problems. Accordingly, there is a need for a device that can instantly determine the physiological symptoms of the user.

### SUMMARY OF THE DISCLOSURE

The disclosure provides a method for recognizing physiological symptoms and a physiological symptom sensing system, which can determine the physiological symptoms of users in time.

The method for recognizing physiological symptoms of the disclosure is provided to recognizing the physiological state of a living body, including: obtaining a sound signal; capturing multiple signal segments from the sound signal; recognizing the sound type corresponding to each of the signal segments; and determining the physiological state of the living body according to the sound type of the signal segment and the combination of the sound type.

In an embodiment of the disclosure, the step of capturing signal segments from the sound signal includes: detecting the sound start point and the sound end point of each of the signal segments from the sound signal.

In an embodiment of the disclosure, the step of recognizing the sound type corresponding to each of the signal segments includes: extracting a plurality of feature values from each of the signal segments; and inputting these feature values into a recognition model to obtain the sound type corresponding to each of the signal segments.

In an embodiment of the disclosure, the step of obtaining the sound signal includes: obtaining the sound signal through a sound sensor provided on a wearable device.

In an embodiment of the disclosure, the method for recognizing physiological symptoms further includes: obtaining movement information of the living body through a motion sensor set on the wearable device; and generating an alert notification based on the movement information.

In an embodiment of the disclosure, the wearable device is a waist protector to obtain the sound signal of the abdomen of the living body.

In an embodiment of the disclosure, the method for recognizing physiological symptoms further includes: recognizing an abnormal situation according to one or more combinations of the sound types.

In an embodiment of the disclosure, the abnormal situation corresponds to a first combination, and the first combination includes silence, an abdominal noise, a bulging sound, spasm, and silence.

In an embodiment of the disclosure, the sound type is one of silence, a grunting sound, spasm, a low-pitched sound, a bulging sound, an abdominal noise, throbbing, hunger, and an emission noise.

The physiological symptom sensing system of the disclosure includes: a sound sensor for sensing sound signals; and a processor coupled to the sound sensor. The processor is configured to: receive a sound signal; capture a plurality of signal segments from the sound signal; recognize the sound type corresponding to each of the signal segments; determine the physiological state of the living body according to the sound type of the signal segment and the combination of the sound type.

Based on the above, the disclosure utilizes the multiple sound types included in the sound signal and the combination of the sound types to further determine the physiological symptoms of the user.

### BRIEF DESCRIPTION OF THE DRAWINGS

FIG. 1 is a block diagram of a physiological symptom sensing system according to an embodiment of the disclosure.
FIG. 2 is a flowchart of a method for recognizing physiological symptoms according to an embodiment of the disclosure.
FIG. 3 is a schematic diagram of a physiological symptom sensing system according to an embodiment of the disclosure.

### DESCRIPTION OF EMBODIMENTS

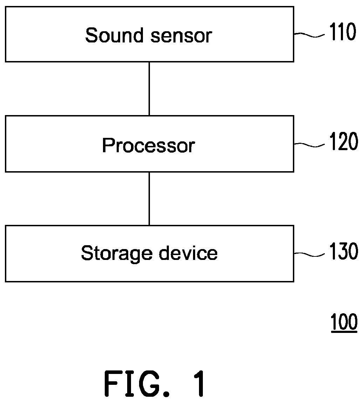

FIG. 1 is a block diagram of a physiological symptom sensing system according to an embodiment of the disclosure. Please refer to FIG. 1, the physiological symptom sensing system 100 includes a sound sensor 110, a processor 120 and a storage device 130.

The sound sensor 110 is, for example, a simple microphone, which can be configured to detect the sound intensity of the environment to generate a sound signal. Here, the sound sensor 110 is configured near the abdomen of the living body to detect the sound generated by the stomach. For example, the sound sensor 110 is provided on the wearable device, and the wearable device is worn on the waist (near the abdomen) of the living body.

The processor 120 is, for example, a central processing unit (CPU), a physical processing unit (PPU), a programmable microprocessor, an embedded control chip, a digital signal processor (DSP), application specific integrated circuits (ASIC) or other similar devices.

The storage device 130 is, for example, any type of fixed or removable random access memory (RAM), read-only memory (ROM), flash memory, hard disk or other similar devices or a combination of these devices. A plurality of code segments are stored in the storage device 130, and the foregoing code segments are executed by the processor 120 after being installed, so as to realize the following physiological symptom recognizing method.

In an embodiment, the processor 120 and the storage device 130 may be disposed together with the sound sensor 110 in the same wearable device. In another embodiment, the physiological symptom sensing system 100 may further include a wearable device provided with a sound sensor 110, as well as another independent electronic device (provided with the processor 120 and the storage device 130 in FIG. 1). The wearable device is worn on the living body to obtain the sound signal of a specific part (such as the abdomen), and then the obtained sound signal is transmitted to an electronic device (such as a smart phone) for analysis. The transmission between the electronic device and the wearable device can be performed, for example, through a short-range wireless communication technology such as Bluetooth.

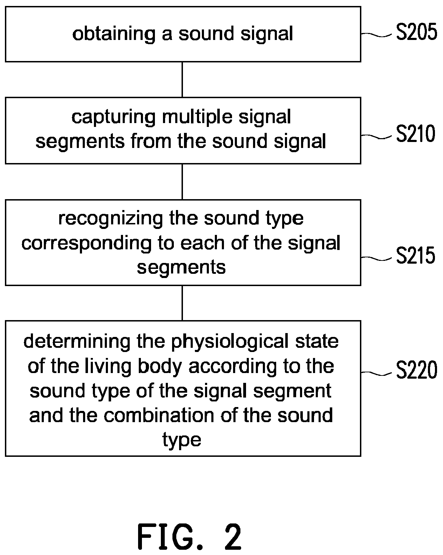

FIG. 2 is a flowchart of a method for recognizing physiological symptoms according to an embodiment of the disclosure. Please refer to FIG. 1 and FIG. 2, in step S205, the sound signal is obtained through the sound sensor 110. After obtaining the sound signal, the sound sensor 110 transmits the sound signal to the processor 120 for analysis and processing.

In step S210, the processor 120 captures multiple signal segments from the sound signal. The processor 120 detects the sound start point and sound end point of each of the signal segments from the sound signal. Since the process of changing sound from silence to sound requires a period of observation before it can be determined accurately, a group of buffering zones is designed as the criterion for judgment. For example, a number of buffering zones are designed to store sound signals according to the receiving order. When the sound energy in the B number of buffering zones in the A number of buffering zones exceeds a preset threshold, the time point of the first buffering zone is used as the sound start point. Specifically, A can be set according to the situation, and B is less than A. Here, A=8 and B=6. In addition, afterwards, the received sound signals are continuously stored in other groups of buffering zones according to the receiving order. When the sound energy in all buffering zones in other groups of buffering zones is lower than a silence threshold, the time point of the first buffering zone in this group of buffering zone is used as the sound end point, and so forth, thereby obtaining a plurality of signal segments.

In addition, End-point Detection (EPD) can also be adopted to find the sound start point and sound end point.

Next, in step S215, the processor 120 recognizes the sound type corresponding to each signal segment. The processor 120 extracts a plurality of feature values from each of the signal segments, and inputs these feature values into the recognition model to obtain the sound type corresponding to each of the signal segments. The recognition model is, for example, a Hidden Markov Model (HMM).

Here, Mel-Frequency Cepstrum Coefficients (MFCC) can be adopted to extract multiple feature values. MFCC is a set of key coefficients used to build Mel-Frequency Cepstrum. The extraction of MFCC feature values includes pre-emphasis processing, frame blocking, window processing, Fourier transform processing, and filter processing.

Pre-emphasis processing includes: The sound signal is set to pass through a high-pass filter H(Z) to enhance the high frequency part, so that the frequency spectrum of the sound signal becomes flat and remains in the entire frequency band from low to high frequency. The same noise ratio can be adopted to obtain the frequency spectrum. Specifically, H(Z)=1-µz⁻¹, where the value of µ is between 0.9 and 1.0, normally it is set that µ=0.97.

Framing blocking includes: N sampling points are assembled into one observation unit, which is called a frame with a size range of approximately 20 to 40 milliseconds. There is an overlap area between two consecutive frames, and the overlap area contains M sampling points. Here, the overlap range is set to 50% (+/-10%) of N. Specifically, the time length of the frame T=sampling frequency F/sampling point N. For example, if the sampling frequency is 8 KHz and the sampling point is 200, the time length of the frame is 200/8000×1000 = 25 ms. Generally, the time length of the frame is set to 25 ms and a step of 10 ms, that is, the overlap area is 15 ms.

Window processing includes: Each frame is multiplied by a window to increase the continuity between the left and right ends of the frame. The Hamming Window is adopted in the disclosure.

Fourier transform processing includes: After window processing is performed, each frame must be subjected to fast Fourier transform to obtain the energy distribution on the frequency spectrum. For example, short-time Fourier transform (STFT) is adopted in the disclosure.

A Fourier transform can be performed to calculate the spectrum on each frame, which is also called short-time Fourier transform (STFT), where the sampling point N is typically 256 or 512, and the Fourier coefficient NFFT is 512.

Filter processing includes: The spectral energy is multiplied by a set of Mel-scale triangular bandpass filters to obtain the log energy output by each filter. Generally speaking, the basic feature vector of each frame has 13 dimensions, including 1 logarithmic energy and 12 cepstral parameters. In addition, Delta Cepstrum coefficients can be further added to display the change of the Cepstrum coefficients with respect to time, thereby adjusting the dimension of the feature vector.

After obtaining multiple feature values, a recognition model such as HMM is further utilized to obtain the sound type corresponding to each of the signal segments. The sound type is, for example, silence, a grunting sound, spasm, a low-pitched sound, a bulging sound, an abdominal noise, or an emission noise. In the actual recognition process, all pronunciations can be more explicitly divided into more basic fundamental pronunciation units, such as phoneme, so as to distinguish the smallest sound units of different pronunciations, and to build an acoustic model based on the phonemes. For example, different phonemes can be established according to different states of the stomach, and different phonemes can be utilized to establish an acoustic model.

The processor 120 utilizes the Hidden Markov Model (HMM) to compare the obtained feature qualities (for example, MFCC coefficients) with the template of the database, thereby calculating the magnitude of the two similar sounds and determining the recognition result.

Finally, in step S220, the processor 120 determines the physiological state of the living body based on the sound type of the signal segment and the combination of the sound type. The processor 120 may determine the corresponding context according to the recognized sound type and the combination thereof. For example, the processor 120 can compare the acquired sound type and the combination thereof with the multiple context data stored in the database to determine the physiological state of the living body (for example, the user). In addition, the normal situation and the abnormal situation can be further distinguished according to the combination of sound types, and an alert notification will be issued when the situation is determined to be an abnormal situation.

**Table 1 shows a combination of the sound types included in different normal situations.**

| Normal situation | Combination of sound types |
|---|---|
| After eating | Silence → grunting → spasm → grunting→ silence |
| After eating | Silence→spasm→bulging→ spasm→ silence |
| Before eating | Silence→ spasm→ abdominal noise → low-pitch sound→ silence |

**Table 2 shows a combination of sound types included in different abnormal situations.**

| Abnormal situation | Combination of sound types |
|---|---|
| After eating-stomach ulcer | Silence→abdominal noise→spasm→silence |
| | Silence→abdominal noise→bulging→spasm→silence |
| | Silence→ abdominal noise→ hunger → spasm→ silence |
| Before eating-duodenal ulcer | Silence→ throbbing → hunger → silence |
| Poor gastrointestinal motility | Silence→gastric water sound→silence |
| | Silence→ intestinal water sound → silence |

For example, referring to Table 2, when the combination of the obtained sound types is silence, an abdominal noise, a bulging sound, spasm, and silence, the situation is determined as an abnormal situation (i.e., after eating-gastric ulcer).

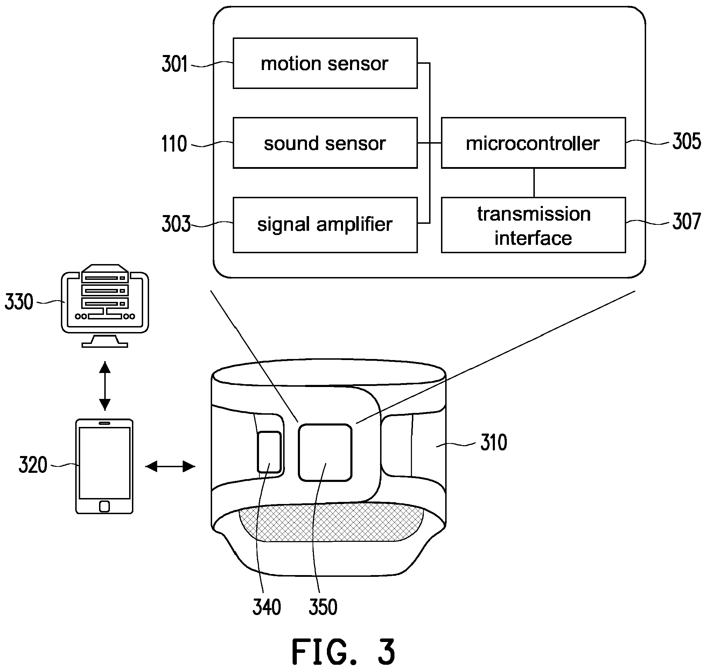

FIG. 3 is a schematic diagram of a physiological symptom sensing system according to an embodiment of the disclosure. The physiological symptom sensing system of this embodiment includes a wearable device 310, a mobile device 320, and a server 330. Here, the wearable device 310 is, for example, a waist protector, and the mobile device 320 is, for example, a smart phone, but the disclosure is not limited thereto.

The wearable device 310 is provided with a battery 340 and a microblock 350. The battery 340 supplies power to each component in the microblock 350. The microblock 350 includes a motion sensor 301, a sound sensor 110, a signal amplifier 303, a microcontroller 305, and a transmission interface 307. The microcontroller 305 is coupled to the motion sensor 301, the sound sensor 110, the signal amplifier 303, and the transmission interface 307. The transmission interface 307 further includes a Wi-Fi module and a Bluetooth module. The Wi-Fi module is configured to perform long-distance transmission and the Bluetooth module is configured to perform short-distance transmission.

In this embodiment, in addition to protecting the waist, the wearable device 310 (waist protector) can also collect the original signal through the sound sensor 110 and enhance the original signal through the signal amplifier 303. The microcontroller 305 utilizes the transmission interface 307 to transmit the processed sound signal to the mobile device 320.

In this embodiment, the mobile device 320 is provided with a processor and a storage device that are not shown, and its functions are the same as those of the processor 120 and the storage device 130 in the embodiment shown in FIG. 1. Please refer to the above description for details. An application program is stored in the storage device and executed by the processor.

In the mobile device 320, the sound signal is analyzed through an application program to detect the physiological state of the living body, and the analyzed data is uploaded to the server 330 for data storage and matching. In addition, the application program can further issue alert notifications when abnormal conditions (such as flatulence, poor gastrointestinal motility, etc.) are detected after analysis. For example, an alert message such as "Relax as appropriate" is displayed on the display of the mobile device 320. In addition, if the application program frequently detects the occurrence of an abnormal state, a message such as "seeking help from a doctor to further determine the cause of symptom is recommended" is displayed on the display of the mobile device 320.

In addition, the wearable device 310 is also equipped with a motion sensor 301 to detect the movement information of the living body wearing the wearable device 310. The motion sensor 301 is, for example, a three-axis sensor. The microcontroller 305 transmits the movement information sensed by the motion sensor 301 to the mobile device 320 through the transmission interface 307 at any time. In the mobile device 320, the movement information of the living body is analyzed through the application program, and an alert notification is generated based on the movement information. For example, if it is detected that the user has been sitting for a long time, the application program can notify the user to get up and walk around by text message or audio message, so it is possible for the user to prevent from sitting in a chair for a long time, such that the burden on the waist can be reduced, thereby avoiding the occurrence of low back pain and decrease of muscle strength as well as weakness of bone and joint.

In addition, a function key can also be provided on the wearable device 310, and the operation of the sound sensor 110 starts to be activated when the function key is enabled. When the function key is enabled again, the operation of the sound sensor 110 is stopped.

In summary, the disclosure utilizes a sound sensor to collect the faint sound generated by a specific part of the user's body, then amplify the sound power, and then collect and analyze these data through algorithm to find out the feature value in order to classify possible conditions, and compare them with the training data stored in the database. If an abnormal situation occurs, an alert notification is issued to notify the user. In this manner, the user's physiological symptoms can be monitored from time to time in daily life, and symptoms can be found before the abnormal state becomes serious and treated as soon as possible.

## Claims

1. A method for recognizing physiological symptoms, configured to recognize a physiological state of a living body, comprising:
obtaining a sound signal;
capturing a plurality of signal segments from the sound signal;
recognizing a sound type corresponding to each of the plurality of signal segments; and
determining the physiological state of the living body according to the sound type of the plurality of signal segments and a combination of the sound type.

2. The method for recognizing physiological symptoms according to claim 1, wherein the step (S205, S210, S215, S220) of capturing the plurality of signal segments from the sound signal comprises:
detecting a sound start point and a sound end point of each of the plurality of signal segments from the sound signal.

3. The method for recognizing physiological symptoms according to claim 1 or 2, wherein the step (S205, S210, S215, S220) of recognizing the sound type corresponding to each of the plurality of signal segments comprises:
extracting a plurality of feature values from each of the plurality of signal segments; and
inputting the plurality of feature values into a recognition model to obtain the sound type corresponding to each of the plurality of signal segments.

4. The method for recognizing physiological symptoms according to one of claims 1 to 3, wherein the step (S205, S210, S215, S220) of obtaining the sound signal comprises:
obtaining the sound signal through a sound sensor (110) provided on a wearable device (310).

5. The method for recognizing physiological symptoms according to claim 4, further comprising:
obtaining movement information of the living body through a motion sensor (301) set on the wearable device (310); and
generating an alert notification based on the movement information.

6. The method for recognizing physiological symptoms according to claim 4 or 5, wherein the wearable device (310) is a waist protector to obtain the sound signal of an abdomen of the living body.

7. The method for recognizing physiological symptoms according to one of claims 1 to 6, further comprising:
recognizing an abnormal situation according to one or more combinations of the sound types.

8. The method for recognizing physiological symptoms according to claim 7, wherein the abnormal situation corresponds to a first combination, and the first combination comprises silence, an abdominal noise, a bulging sound, spasm, and silence.

9. The method for recognizing physiological symptoms according to one of claims 1 to 8, wherein the sound type is one of silence, a grunting sound, spasm, a low-pitched sound, a bulging sound, an abdominal noise, throbbing, hunger, and an emission noise.

10. A physiological symptom sensing system (100), comprising:
a sound sensor (110) configured to obtain a sound signal;
a processor (120) coupled to the sound sensor (110), wherein the processor (120) is configured to:
receive the sound signal;
capture a plurality of signal segments from the sound signal;
recognize a sound type corresponding to each of the plurality of signal segments;
determine a physiological state of a living body according to the sound type of the plurality of signal segments and a combination of the sound type.

11. The physiological symptom sensing system (100) according to claim 10, further comprising:
a wearable device (310) provided with the sound sensor (110), and further comprising:
a transmission interface (307); and
a signal amplifier (303) configured to amplify an original signal sensed by the sound sensor (110) to obtain the sound signal, and then transmit the sound signal to the processor (120) through the transmission interface (307).

12. The physiological symptom sensing system (100) according to claim 11, wherein the wearable device (310) is a waist protector to obtain the sound signal of an abdomen of the living body, and the wearable device (310) further comprises:
a motion sensor (301) detecting movement information of the living body wearing the wearable device (310), and transmitting the movement information to the processor (120) through the transmission interface (307),
wherein the processor (120) generates an alert notification based on the movement information.

13. The physiological symptom sensing system (100) according to one of claims 10 to 12, wherein the processor (120) is configured to:
detect a sound start point and a sound end point of each of the plurality of signal segments from the sound signal.

14. The physiological symptom sensing system (100) according to one of claims 10 to 13, wherein the processor (120) is configured to:
extract a plurality of feature values from each of the plurality of signal segments; and
input the plurality of feature values into a recognition model to obtain the sound type corresponding to each of the plurality of signal segments.

15. The physiological symptom sensing system (100) according to claim 10, wherein the sound type is one of silence, a grunting sound, spasm, a low-pitched sound, a bulging sound, an abdominal noise, throbbing, hunger, and an emission noise,
an abnormal situation is recognized according to one or more combinations of the sound types,
the abnormal situation corresponds to a first combination, and the first combination comprises silence, an abdominal noise, a bulging sound, spasm, and silence.

## Amended claims

### Amended claims in accordance with Rule 137(2) EPC.

1. A method for recognizing physiological symptoms, configured to recognize a physiological state of a living body, comprising:
obtaining a sound signal by a sound sensor (110);
**characterized in that** the method further comprises:
capturing a plurality of signal segments from the sound signal by a processor (120);
using a recognition model to recognize a sound type corresponding to each of the plurality of signal segments by the processor (120); and
determining the physiological state of the living body by the processor (120) according to the sound types of the plurality of signal segments and a combination of the sound types, wherein the physiological state is a state of a normal situation or a state of an abnormal situation.

2. The method for recognizing physiological symptoms according to claim 1, wherein the step (S205, S210, S215, S220) of capturing the plurality of signal segments from the sound signal by the processor (120) comprises:
detecting a sound start point and a sound end point of each of the plurality of signal segments from the sound signal.

3. The method for recognizing physiological symptoms according to claim 1 or 2, wherein the step (S205, S210, S215, S220) of using the recognition model to recognize the sound type corresponding to each of the plurality of signal segments by the processor (120) comprises:
extracting a plurality of feature values from each of the plurality of signal segments; and
inputting the plurality of feature values into the recognition model to obtain the sound type corresponding to each of the plurality of signal segments.

4. The method for recognizing physiological symptoms according to one of claims 1 to 3, wherein the sound sensor (110) is provided on a wearable device (310).

5. The method for recognizing physiological symptoms according to claim 4, further comprising:
obtaining movement information of the living body through a motion sensor (301) set on the wearable device (310); and
generating an alert notification based on the movement information by a processor (120).

6. The method for recognizing physiological symptoms according to claim 4 or 5, wherein the wearable device (310) is a waist protector to obtain the sound signal of an abdomen of the living body.

7. The method for recognizing physiological symptoms according to one of claims 1 to 6, further comprising:
recognizing an abnormal situation according to one or more combinations of the sound types by the processor (120).

8. The method for recognizing physiological symptoms according to claim 7, wherein the abnormal situation corresponds to at least one combination, and the at least one combination comprises silence, an abdominal noise, a bulging sound, spasm, and silence.

9. The method for recognizing physiological symptoms according to one of claims 1 to 8, wherein the sound type is one of silence, a grunting sound, spasm, a low-pitched sound, a bulging sound, an abdominal noise, throbbing, hunger, and an emission noise.

10. A physiological symptom sensing system (100), comprising:
a sound sensor (110) configured to obtain a sound signal;
**characterized in that** the physiological symptom sensing system (100) further comprises:
a processor (120) coupled to the sound sensor (110), wherein the processor (120) is configured to:
receive the sound signal;
capture a plurality of signal segments from the sound signal;
recognize a sound type corresponding to each of the plurality of signal segments;
determine a physiological state of a living body according to the sound types of the plurality of signal segments and a combination of the sound types, wherein the physiological state is a state of a normal situation or a state of an abnormal situation.

11. The physiological symptom sensing system (100) according to claim 10, further comprising:
a wearable device (310) provided with the sound sensor (110), and further comprising:
a transmission interface (307); and
a signal amplifier (303) configured to amplify an original signal sensed by the sound sensor (110) to obtain the sound signal, and then transmit the sound signal to the processor (120) through the transmission interface (307).

12. The physiological symptom sensing system (100) according to claim 11, wherein the wearable device (310) is a waist protector to obtain the sound signal of an abdomen of the living body, and the wearable device (310) further comprises:
a motion sensor (301) detecting movement information of the living body wearing the wearable device (310), and transmitting the movement information to the processor (120) through the transmission interface (307),
wherein the processor (120) generates an alert notification based on the movement information.

13. The physiological symptom sensing system (100) according to one of claims 10 to 12, wherein the processor (120) is configured to:
detect a sound start point and a sound end point of each of the plurality of signal segments from the sound signal.

14. The physiological symptom sensing system (100) according to one of claims 10 to 13, wherein the processor (120) is configured to:
extract a plurality of feature values from each of the plurality of signal segments; and
input the plurality of feature values into a recognition model to obtain the sound type corresponding to each of the plurality of signal segments.

15. The physiological symptom sensing system (100) according to claim 10, wherein the sound type is one of silence, a grunting sound, spasm, a low-pitched sound, a bulging sound, an abdominal noise, throbbing, hunger, and an emission noise,
an abnormal situation is recognized according to one or more combinations of the sound types,
the abnormal situation corresponds to at least one combination, and the at least one combination comprises silence, an abdominal noise, a bulging sound, spasm, and silence.
